# EUROPÄISCHE PATENTSCHRIFT

(11) **EP 0 561 295 B1**
(45) Veröffentlichungstag und Bekanntmachung des Hinweises auf die Patenterteilung: **15.05.1996**
(21) Anmeldenummer: 93104033.1
(22) Anmeldetag: 12.03.1993
(51) Int. Cl.: A61B 17/58

(54) **Aussenbefestigter Marknagel**
Externally fastened intramedullary nail
Clou intramédullaire à fixation externe

(30) Priorität: 20.03.1992 DE 4209122
(43) Veröffentlichungstag der Anmeldung: 22.09.1993
(73) Patentinhaber: Kessler, Sigurd, Dr., D-82110 Germering (DE)
(72) Erfinder: Kessler, Sigurd, Dr., D-82110 Germering (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- EP-A- 0 029 752
- EP-A- 0 095 990
- EP-A- 0 381 462
- DE-A- 2 821 785
- DE-A- 4 002 400
- DE-B- 1 054 659
- DE-U- 8 330 389
- DE-U- 8 809 715
- FR-A- 2 141 020
- FR-A- 2 243 673
- FR-A- 2 484 243

## Beschreibung

Die Erfindung betrifft einen Marknagel zur intramedullären Anwendung bei der Heilung von Knochenbeschädigungen, insbesondere von Frakturen von Röhrenknochen, der in eine Markhöhle des Knochens einsetzbar sowie an beiden Enden mit Fixiermitteln am Knochen befestigbar ist, wobei ein Ende des Marknagels als an der Außenseite des Knochens mittels Schrauben fixierbares Befestigungsteil und der Marknagel als Hohlnagel ausgebildet ist, der einen an beiden Enden offenen Innenraum besitzt.

Nägel dieser Art werden üblicherweise bei der Behandlung von Röhrenknochen eingesetzt, wenn entweder davon auszugehen ist, daß die Bruchstelle durch eine bloße Ruhigstellung nicht oder nicht gut genug zusammenwächst oder eine lange Ruhigstellung, die in der Regel 8 bis 12 Wochen beträgt, nicht in Kauf genommen werden kann.

Aus der FR-A-2 484 243 ist ein Nagel der eingangs genannten Art bekannt (vgl. Oberbegriff der Ansprüche 1 und 2). Der Nagel besteht aus einem inneren und einem äußeren jeweils im wesentlichen rohrförmigen Element, von denen das innere Element in dem äußeren Element verschiebbar angeordnet ist und an einem Ende Lamellen zur Fixierung innerhalb der Markhöhle des Knochens aufweist. Das äußere Element ist über eine separate Befestigungsplatte, welche an dem außerhalb des Knochens gelegenen Ende des äußeren Elements befestigbar ist, über Schrauben an der Außenseite des Knochens fixierbar.

Aus der DE-OS 40 02 400 ist ein Nagel bekannt, der in die Markhöhle des Knochens eingesetzt und beidseits der Knochenbeschädigung mittels Querbolzen am Knochen fixiert wird. Die Querbolzen sind nach dieser Offenlegungsschrift als Knochenschrauben ausgebildet. Zur Verbindung der Knochenschrauben mit dem in der Markhöhle eingesetzten Marknagel muß der Knochen beidseitig der Bruchstelle angebohrt werden.

Um die korrekte Positionierung der Schrauben in die Löcher gewährleisten zu können, müssen die Bohrungen mittels aufwendiger Zielvorrichtungen oder unter Röntgen-Beobachtung durchgeführt werden.

Frakturen, die sehr nahe an einem Ende des Knochens liegen, können mit diesen Nägeln nicht behandelt werden, da eine Fixierung beidseits der Bruchstelle nicht möglich ist.

Es sind auch Marknägel bekannt, bei denen auf eine Fixierung des Nagels über die Querbolzen verzichtet wird. Bei diesen Nägeln wird die Fixierung des Nagels im Knochen beispielsweise dadurch erreicht, daß der Nagel eine scharfkantige Spitze aufweist, die am Ende der Markhöhle in das Knochenmaterial getrieben wird. Diese Verbindung weist jedoch keine große Haltbarkeit auf und lockert sich durch die Bewegungen während des Tragens üblicherweise bereits nach einigen Tagen.

Aus der EP-A 0 381 462 ist ein Knochennagel bekannt, der als Hohlnagel ausgebildet ist. Ein Ende des Nagels kann über ein Befestigungsteil am Knochen fixiert werden. Dazu wird der Nagel zusammen mit dem Befestigungsteil über eine Bohrung in die Markhöhle des Knochens eingeführt. Anschließend wird das innerhalb des Knochens gelegene Befestigungsteil mittels Schrauben mit der Innenwand der Bohrung verschraubt. Eine Befestigung des Nagels an der Außenseite des Knochens findet nicht statt.

Nachdem der Nagel über dieses Befestigungsteil in seiner gewünschten Position im Knochen mit einem Ende fixiert ist, muß das gegenüberliegende Ende des Nagels auf herkömmliche Weise durch als Knochenschrauben ausgebildete Querbolzen fixiert werden. Die für die Querbolzen benötigten Bohrungen müssen wie bei einem Nagel nach der DE-OS 40 02 400 mittels aufwendiger Zielvorrichtungen oder unter Röntgen-Beobachtung durchgeführt werden.

Aus der EP-0 095 990 ist ein Knochennagel bekannt, der über ein Befestigungsteil von außen am Knochen befestigbar ist. Nach Einführen dieses als Vollnagel ausgebildeten Knochennagels in die Markhöhle verbleibt ein Ende des Nagels außerhalb des Knochens und wird an dessen Außenseite über eine Schraube befestigt. Das andere Ende des Nagels weist ein Spitze auf, die am Ende der Markhöhle in das Knochenmaterial getrieben wird. Diese Verbindung weist jedoch keine große Haltbarkeit auf und lockert sich durch die Bewegungen während des Tragens üblicherweise bereits nach einigen Tagen.

Aufgabe der Erfindung ist es, einen Marknagel zur intramedullären Anwendung bei der Heilung von Knochenbeschädigungen so auszubilden, daß eine einfache und zugleich sichere Verankerung des Marknagels im Knochen gewährleistet ist und eine maximale Ausdehnung der zu versorgenden Knochenstrecke ermöglicht wird. Dabei soll sowohl eine statische als auch eine dynamische Verriegelung zur Behandlung von Schräg- bzw. Querfrakturen möglich sein.

Gelöst wird diese Aufgabe nach der Erfindung durch die Merkmale der Patentansprüche 1 oder 2.

Durch die Ausbildung des Marknagels als Hohlnagel und die Fixierung beider Enden des Nagels an der Außenseite des Knochens ist eine besonders einfache und zugleich sichere Verankerung des Nagels im Knochen gewährleistet. Dabei ist durch die von außen zugängliche Schraube das freie Ende des Marknagels im Knochen fixierbar und gleichzeitig, durch die Verwendung der Schraube als Zugschraube ein vorbestimmter Druck zum Zusammenpressen der Bruchflächen einstellbar. Durch diese dynamische Verriegelung wird eine optimale Heilwirkung bei gleichzeitiger minimaler Schmerzentwicklung bei Querfrakturen ermöglicht.

Bevorzugt wird eine Unterlegscheibe vorgesehen, die so ausgebildet ist, daß sie über ihre ganze Fläche an der Außenseite des Knochens anliegt, um dadurch einen sicheren Sitz am Knochen zu gewährleisten.

Nach einer zweckmäßigen Ausführungsform ist das Befestigungsteil als separates Teil ausgebildet, das an einem Ende des Marknagels befestigt, insbesondere verschraubt ist. Dabei können beispielsweise das Befestigungsteil und der Marknagel aneinandergelegt und mittels Schrauben miteinander verbunden werden. Es kann aber auch jeweils ein Ende des Befestigungsteils und des Marknagels so mit einem Gewinde versehen sein, daß die beiden Teile stirnseitig miteinander verschraubt werden können.

Durch diese separate Ausbildung des Befestigungsteils können mehrere Vorteile erreicht werden. Beispielsweise können Marknägel mit verschiedenen Längen mit ein und demselben Befestigungsteil verbunden und verwendet werden. Weiterhin können das Befestigungsteil und der Marknagel so ausgebildet und miteinander befestigt sein, daß sie sowohl für die rechten als auch für die linken Gliedmaßen verwendbar sind. Die Verbindungsstelle zwischen Befestigungsteil und Marknagel kann dabei sowohl innerhalb als auch außerhalb des Knochens liegen.

In einer weiteren Ausführungsform der Erfindung ist das dem Befestigungsteil zugewandte Ende des Marknagels mit verstärkter Wandung als der übrige Teil des Marknagels ausgebildet. Da die Verbindung des Marknagels mit dem Befestigungsteil beispielsweise über eine Verschraubung erfolgt, kann das dem Befestigungsteil zugewandte Ende des Marknagels zumindest eine Bohrung zur Aufnahme der entsprechenden Schraube aufweisen. Wenn sich das dem Befestigungsteil zugewandte Ende des Marknagels bereits außerhalb der Markhöhle befindet, ist es zweckmäßig, dieses Ende breiter als den übrigen Teil des Marknagels auszubilden, um damit die durch die Bohrung verlorene Stabilität des Marknagels durch zusätzliches Material wiederherzustellen oder sogar noch zu übertreffen.

Nach einer weiteren bevorzugten Ausführungsform umfaßt das Befestigungsteil ein Distanzstück, das mit dem Marknagel drehfest verbunden ist. Die drehfeste Verbindung kann dabei beispielsweise durch eine Kronenverzahnung zwischen Distanzstück und Marknagel erreicht werden. Das Distanzstück ist dabei bevorzugt als Hülse ausgebildet, die an einem Ende Ansätze zur Fixierung am Knochen, beispielsweise über Schrauben aufweist. Bevorzugt ist das Distanzstück mit dem Marknagel über eine durch die Hülse verlaufende Schraube verbunden.

Bei einer Fixierung der beiden Enden des Marknagels am Knochen über drehfeste Verbindungen wird eine statische Verriegelung geschaffen, mit der ein Verdrehen bzw. ein Kippen der Knochenteile gegeneinander und damit eine Verkürzungstendenz des Knochens verhindert wird, so daß auch Schräg- oder Trümmerfrakturen behandelt werden können.

Weitere besonders vorteilhafte Merkmale der Erfindung sind in den Unteransprüchen angegeben.

Bei einer bevorzugten Verwendung eines erfindungsgemäßen Marknagels wird der Knochen zumindest auf einer Seite der Bruchstelle zentrisch oder seitlich angebohrt, durch diese erste Bohrung das dem Befestigungsteil gegenüberliegende Ende des Marknagels in die Markhöhle eingeführt und nach vollständiger Einbringung des Marknagels das Befestigungsteil am Knochen fixiert, insbesondere verschraubt.

Durch diese Fixierung des Marknagels von außen am Knochen entfällt die üblicherweise benötigte Zielvorrichtung bzw. das Anbringen von Bohrungen unter Beobachtung am Röntgenschirm, was zu einer wesentlichen Vereinfachung des Fixierens des Marknagels am Knochen führt.

Durch die Fixierung des Marknagels am massiven Knochenende wird gleichzeitig eine sehr stabile Verankerung des Marknagels am Knochen geschaffen.

Bei einer weiteren bevorzugten Verwendung wird der Knochen beidseitig der Bruchstelle angebohrt und das dem Befestigungsteil gegenüberliegende Ende des Marknagels mittels der durch die zweite Bohrung eingeführten Schraube bzw. des Distanzstückes und der Schraube im Knochen fixiert. Dabei wird bevorzugt diese zweite Bohrung seitlich außerhalb der Verlängerung der Markhöhle angebracht, so daß beispielsweise bei Behandlung einer Oberarmfraktur, die zweite Bohrung nicht wie üblich senkrecht von oben durch das Schultergelenk hindurchgeführt wird, sondern schräg von der Seite angesetzt wird. Dadurch wird eine Bohrung durch die Sehnen verhindert, wodurch Verletzungen der Sehnen und dadurch verursachte langwierige Rehabilitationsprozesse vermieden werden.

Nach einer anderen bevorzugten Verwendung wird vor Einbringung des Marknagels ein Führungsdraht so in den Knochen eingeführt, daß dessen beide Enden durch die erste bzw. zweite Bohrung nach außen heraustreten. Der hohle Marknagel wird über den Führungsdraht durch die erste Bohrung und die hohle Schraube bzw. das Distanzstück und die Schraube über den Führungsdraht durch die zweite Bohrung in den Knochen eingebracht, wodurch eine besonders einfache Verschraubung des Marknagels mit der Schraube ermöglicht wird.

Nach einer weiteren bevorzugten Verwendung werden kurz vor dem vollständigen Einbringen des Marknagels die Spickdrähte so in den Marknagel hineingeschoben, daß sie durch die Austrittsöffnungen in der Abschlußkappe heraustreten und in das Knochenmaterial eindringen. Nach genügendem Eindringen der Spickdrähte in das Knochenmaterial werden die dem Befestigungsteil zugewandten Enden der Spickdrähte an diesem befestigt und anschließend der Marknagel vollständig so in den Knochen eingeführt, daß sich die im Knochenmaterial steckenden Enden der Spickdrähte gegenüber dem Marknagel verkanten und eine im wesentlichen senkrechte Stellung zur Längsrichtung des Knochens einnehmen. Nach vollständiger Einbringung des Marknagels wird das Befestigungsteils am Knochen fixiert.

Bei dieser Verwendung muß der Knochen nur an einer Stelle angebohrt werden, wobei durch das Verkanten der Spickdrähte gegenüber dem Marknagel eine sehr stabile Verankerung des Marknagels im Knochenmaterial erfolgt.

Der Vorteil eines erfindungsgemäß ausgebildeten Marknagels und seiner Verwendung liegt zum einen in der sicheren und stabilen Verankerung des Marknagels mit dem Knochen, wobei diese Fixierung des Marknagels am Knochen sehr einfach von außen vorgenommen werden kann. Zum anderen ist eine maximale Ausdehnung der zu versorgenden Knochenstrecke gewährleistet, da die Bohrungen bis ganz an die Enden des Knochens gelegt werden können.

Darüber hinaus können durch die wahlweise Ausbildung als statische bzw. als dynamische Verriegelung sowohl Schräg- und Trümmer- als auch Querfrakturen behandelt werden. Ein zusätzlicher Vorteil ist, daß über die Zugschraube der Anpreßdruck der Bruchstellen so eingestellt werden kann, daß eine optimale Heilwirkung bei gleichzeitiger minimaler Schmerzbelastung eingestellt werden kann.

Ein weiterer Vorteil der Erfindung ist der modulare Aufbau des Marknagels. Durch die einfache Ausbildung des Marknagels als Röhre mit zwei endseitigen Gewinden, können je nach vorliegendem Verletzungsfall die unterschiedlichen Aufsätze (Befestigungsteil, Distanzstück, Schraube mit Unterlegscheibe, Abschlußkappe) optimal miteinander kombiniert werden. Darüber hinaus wird die Menge der üblicherweise im oder in unmittelbarer Nähe des Operationssaals zu lagernden Teile deutlich verringert, wodurch sowohl der Platz als auch die Kosten für die Lagerung reduziert werden können.

Die Erfindung wird nachfolgend anhand eines Ausführungsbeispiels unter Bezugnahme auf die Zeichnung erläutert; in dieser zeigt:
- Fig. 1: eine Seitenansicht eines Oberarmknochens im Teilschnitt mit einem erfindungsgemäß ausgebildeten Marknagel,
- Fig. 2: eine Vorderansicht eines Oberarmknochens im Teilschnitt mit einem erfindungsgemäß ausgebildeten Marknagel,
- Fig. 3: das obere Ende eines Oberarmknochens mit einem erfindungsgemäß ausgebildeten, über ein Distanzstück befestigten Marknagel,
- Fig. 4: drei verschieden Stellungen eines erfindungsgemäß ausgebildeten Marknagels während des Einsetzvorganges in einen Knochen,
- Fig. 5: die Befestigung zweier Spickdrähte an einem erfindungsgemäß ausgebildeten Befestigungsteil des Marknagels und
- Fig. 6: einen über ein Distanzstück und Spickdrähten befestigten erfindungsgemäß ausgebildeten Marknagel.

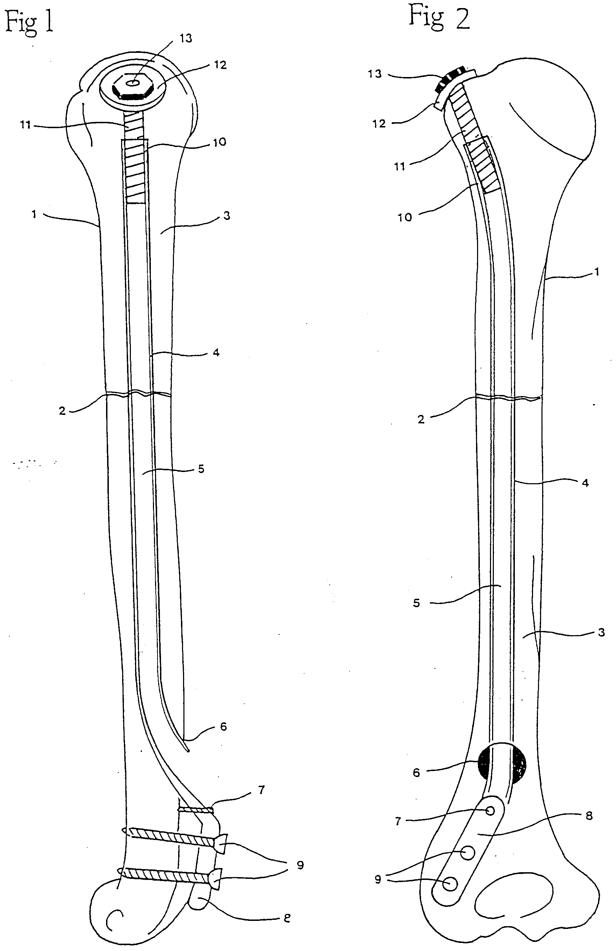

Fig. 1 zeigt einen Knochen 1 mit einer Bruchstelle 2, in dessen Markhöhle 3 ein erfindungsgemäß ausgebildeter Marknagel 4 eingesetzt ist. Der Marknagel 4 ist als Hohlnagel ausgebildet, der einen an beiden Enden offenen Innenraum 5 besitzt. Bei dem in Fig. 1 dargestellten Knochen 1 handelt es sich um einen Oberarmknochen mit Querfraktur.

Das untere Ende des Marknagels tritt durch eine erste Bohrung 6 seitlich am unteren Ende des Knochens 1 aus und ist über eine Schraube 7 mit einem ersten Befestigungsteil 8 verbunden. Das Befestigungsteil 8 liegt an der Außenseite des Knochens 1 an und ist mittels Schrauben 9 an diesem fixiert.

Im oberen Ende des Marknagels 4 ist ein Innengewinde 10 ausgebildet, in das eine Schraube 11 eingeschraubt ist. Der Kopf der Schraube 11 liegt dabei über eine Unterlegscheibe 12 an der Außenseite des oberen Endes des Knochens 1 an. Die Schraube 11 ist als Hohlschraube ausgebildet und weist in ihrem Kopf eine Öffnung 13 auf.

Fig. 2 zeigt eine Vorderansicht der Darstellung aus Fig. 1. Man erkennt hieraus, daß die Schraube 11 über eine zweite Bohrung 14 in den Knochen 1 eindringt und die Unterlegscheibe 12 verhindert, daß der Kopf der Schraube 11 in die zweite Bohrung 14 einbricht. Die Unterlegscheibe 12 ist so ausgebildet, daß sie über ihre ganze Fläche an der Außenseite des Knochens 1 anliegt.

Der erfindungsgemäße Marknagel 4 nach Fig. 1 und 2 bildet eine dynamische Verriegelung, mit der vorzugsweise Querfrakturen behandelt werden können.

Zum Einbringen des Marknagels 4 in die Markhöhle 3 des Knochens 1 wird der Knochen 1 zunächst so an seinem unteren und oberen Ende angebohrt, daß die Markhöhle 3 an ihrem unteren Ende seitlich über die erste Bohrung 6 und an ihrem oberen Ende seitlich über die zweite Bohrung 14 nach außen geöffnet ist. Anschließend wird durch die beiden Bohrungen 6, 14 ein Führungsdraht in den Knochen 1 eingeführt.

Der Marknagel 4 wird durch die erste Bohrung 6 über den Führungsdraht von unten in die Markhöhle 3 eingeführt, bis das Befestigungsteil 8 an der unteren Außenseite des Knochens 1 anliegt.

Die Schraube 11 wird über den Führungsdraht durch die zweite Bohrung 14 geführt und über das Innengewinde 10 des Marknagels 4 mit diesem verschraubt. Die Verschraubung wird dabei so festgezogen, daß eine optimale Verbindung der Knochenstücke an der Bruchstelle 2 erfolgt.

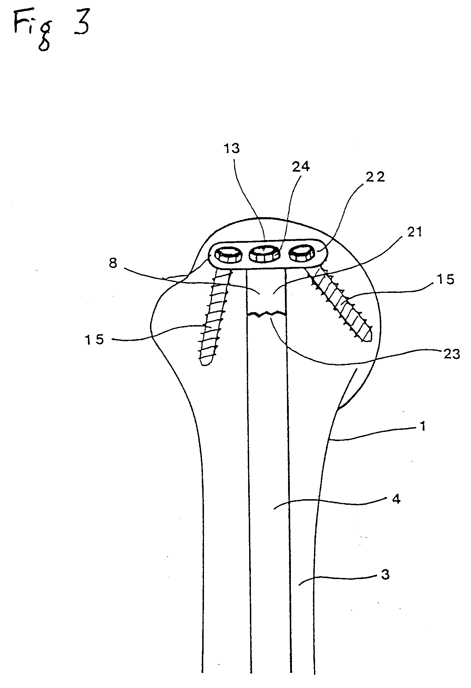

In Fig. 3 ist am oberen Ende des Marknagels 4 anstelle der Unterlegscheibe 12 ein Distanzstück 21 angeordnet. Das Distanzstück 21 ist über Ansätze 22 mit Schrauben 15 am Knochen 1 befestigt und weist an seinem unteren Ende eine Kronenverzahnung 23 auf. Diese Kronenverzahnung 23 greift in eine entsprechende Verzahnung am Ende des Marknagels 4 ein und verhindert somit eine Verdrehung des Distanzstückes 21 gegenüber dem Marknagel 4.

Das Distanzstück 21 ist als Hülse ausgebildet und über eine Schraube 24, die durch die Hülse verläuft, mit dem Marknagel 4 verschraubt. Die Schraube 24 ist wie die Schraube 11 in Fig. 1 und 2 als Hohlschraube ausgebildet und weist in ihrem Kopf eine Öffnung 13 auf.

Zum Fixieren des Marknagels 4 werden sowohl das Distanzstück 21 als auch die Schraube 24 analog zur Schraube 11 nach Fig. 1 und 2 über einen Führungsdraht durch die zweite Bohrung 14 geführt.

Durch Verwendung des Distanzstückes 21 wird eine statische Verriegelung geschaffen, mit der Schräg- und Trümmerfrakturen bzw. Frakturen mit Verkürzungstendenz behandelt werden können, da durch die beidseitige, positionsfeste Fixierung des Marknagels 4 ein Verschieben, Verdrehen und Kippen der Knochenfragmente gegeneinander verhindert wird.

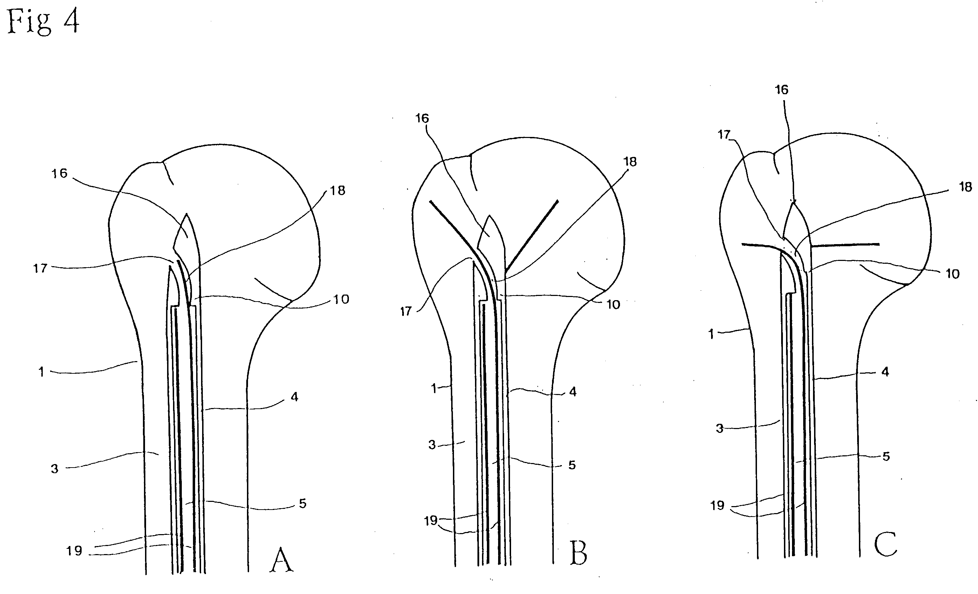

Fig. 4 zeigt eine weitere Ausführungsform eines erfindungsgemäß ausgebildeten Marknagels 4. Dabei ist eine als Spitze ausgebildete Abschlußkappe 16 mit dem Marknagel 4 über das Innengewinde 10 verschraubt. Die Abschlußkappe 16 weist Austrittsöffnungen 17 auf, die über Kanäle 18 mit dem Innenraum 5 des Marknagels 4 in Verbindung stehen.

Im Innenraum 5 des Marknagels 4 sind Spickdrähte 19 angeordnet, deren der Anschlußkappe 16 zugewandten Enden über die Kanäle 18 durch die Austrittsöffnungen 19 verschiebbar sind.

In Fig. 4 a sind die Spickdrähte vollständig im Marknagel 4 und in den Kanälen 18 angeordnet, während in den Fig. 4 b und 4 c die Spickdrähte durch die Austrittsöffnungen 17 aus dem Marknagel 4 herausverschoben sind.

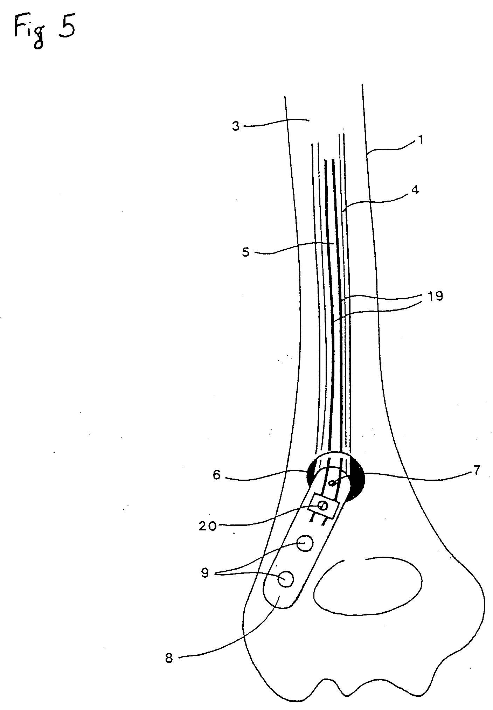

In Fig. 5 sind die unteren Enden der Spickdrähte 19 über eine Klemmschraube 20 an dem Befestigungsteil 8 befestigt.

Zum Einbringen eines Marknagels 4 gemäß der Ausführungsform nach den Fig. 4 und 5 werden die Spickdrähte 19 kurz vor dem vollständigen Einbringen des Marknagels 4, d. h. bei Erreichen der Stellung des Marknagels 4 nach Fig. 4 a, so von unten in den Marknagel 4 hineingeschoben, daß sie durch die Austrittsöffnungen 17 in der Abschlußkappe 16 heraustreten und in das Knochenmaterial eindringen (Fig. 4 b).

Nach genügendem Eindringen der Spickdrähte 19 in das Knochenmaterial (Fig. 4 b) werden die unteren Enden der Spickdrähte 19 über die Klemmschraube 20 an dem Befestigungsteil 8 fixiert.

Anschließend wird der Marknagel 4 vollständig so in den Knochen 1 eingeführt, daß sich die im Knochenmaterial steckenden Enden der Spickdrähte 19 gegenüber dem Marknagel 4 verkanten und eine im wesentlichen senkrechte Stellung zur Längsrichtung des Knochens 1 einnehmen (Fig. 4 c). Nach dem vollständigen Einbringen des Marknagels 4 wird das Befestigungsteil 8 am Knochen 1 mit den Schrauben 9 fixiert.

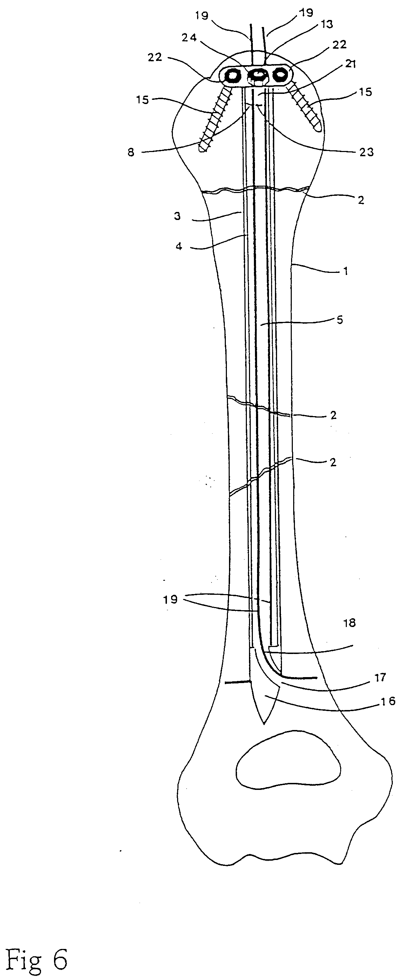

Fig. 6 zeigt einen Knochen 1 mit mehreren Bruchstellen 2, die teils schräg verlaufen, teils sehr dicht am Knochenende gelegenen sind. In den Knochen 1 ist ein erfindungsgemäßer Marknagel 4 eingesetzt, der an seinem oberen Ende über das Distanzstück 21 und an seinem unteren Ende über die Spickdrähte 19 am Knochen 1 fixiert ist. Die Spickdrähte 19 können mit einer nicht dargestellten Klemmschraube 20 analog zu Fig. 5 am Distanzstück 21 befestigt werden.

Durch diese Kombination von Befestigungsarten wird eine statische Verriegelung geschaffen, mit der bevorzugt Schräg- und Trümmerfrakturen behandelt werden können. Zusätzlich kann durch Einführen des Marknagels 4 von oben auch die sehr dicht am Knochenende gelegene Bruchstelle 2 versorgt werden.

### Bezugszeichenliste

- 1: Knochen
- 2: Bruchstelle
- 3: Markröhre
- 4: Marknagel
- 5: Innenraum
- 6: erste Bohrung
- 7: Schraube
- 8: Befestigungsteil
- 9: Schrauben
- 10: Innengewinde
- 11: Schraube
- 12: Unterlegscheibe
- 13: Öffnung
- 14: zweite Bohrung
- 15: Schrauben
- 16: Abschlußkappe
- 17: Austrittsöffnung
- 18: Kanäle
- 19: Spickdrähte
- 20: Klemmschraube
- 21: Distanzstück
- 22: Ansätze
- 23: Kronenverzahnung
- 24: Schraube

## Patentansprüche

1. Marknagel zur intramedullären Anwendung bei der Heilung von Knochenbeschädigungen, insbesondere von Frakturen von Röhrenknochen, der in eine Markhöhle (3) des Knochens einsetzbar sowie an beiden Enden mit Fixiermitteln (9, 11) am Knochen (1) befestigbar ist, wobei ein Ende des Marknagels (4) als an der Außenseite des Knochens (1) mittels Schrauben (9, 15) fixierbares Befestigungsteil (8)
und der Marknagel als Hohlnagel (4) ausgebildet ist, der einen an beiden Enden offenen Innenraum (5) besitzt,
dadurch **gekennzeichnet,**
daß das Befestigungsteil (8, 22) von einem Wandungsteil des Hohlnagels (4) oder einem separaten Teil gebildet ist, das mit dem Hohlnagel (4) verbindbar ist, und daß das dem Befestigungsteil (8, 22) gegenüberliegende offene Ende des Hohlnagels (4) ein Innen- oder Außengewinde (10) aufweist, das mit einer Schraube (11) mit Außen- oder Innengewinde (10) verschraubbar ist zur Fixierung des in die Markhöhle (3) eingesetzten Hohlnagels (4) von außen, wobei der Kopf der Schraube (11) insbesondere über eine Unterlegscheibe (12) von außen am Knochen (1) anliegt und dadurch ein Einbrechen des Kopfes in den Knochen (1) verhindert wird.

2. Marknagel (4) zur intramedullären Anwendung bei der Heilung von Knochenbeschädigungen, insbesondere von Frakturen von Röhrenknochen, der in einer Markhöhle (3) des Knochens (1) einsetzbar sowie an beiden Enden mit Fixiermitteln (9, 11) am Knochen (1) befestigbar ist, wobei ein Ende des Marknagels (4) als an der Außenseite des Knochens (1) mittels Schrauben (9, 15) fixierbares Befestigungsteil (8)
und der Marknagel als Hohlnagel (4) ausgebildet ist, der einen an beiden Enden offenen Innenraum (5) besitzt,
dadurch **gekennzeichnet,**
daß das Befestigungsteil (8, 22) von einem Wandungsteil des Hohlnagels (4) oder einem separaten Teil gebildet ist, das mit dem Hohlnagel (4) verbindbar ist, und daß an dem dem Befestigungsteil (8, 22) gegenüberliegenden offenen Ende des Hohlnagels (4) eine als Spitze ausgebildete Abschlußkappe (16) verschraubbar befestigt ist, die vorzugsweise seitliche Austrittsöffnungen (17) aufweist, die bei auf dem Ende des Hohlnagels (4) aufgeschraubter Abschlußkappe (16) über Kanäle (18) mit dem Innenraum (5) des Hohlnagels (4) in Verbindung stehen, in dem Spickdrähte (19) verschiebbar angeordnet sind, deren der Abschlußkappe (16) zugewandte Enden über die Kanäle (18) durch die Austrittsöffnungen (17) verschiebbar sind und deren dem Befestigungsteil (8) zugewandte Enden an diesem befestigbar sind.

3. Marknagel nach Anspruch 2,
dadurch **gekennzeichnet,**
daß die Abschlußkappe (16) zwei Austrittsöffnungen (17) aufweist und im Innenraum (5) des Hohlnagels (4) zwei Spickdrähte (19) angeordnet sind.

4. Marknagel nach Anspruch 2 oder 3,
dadurch **gekennzeichnet,**
daß an dem Befestigungsteil (8) eine insbesondere als Klemmschraube (20) ausgebildete Vorrichtung zum Befestigen der Spickdrähte (19) angeordnet ist.

5. Marknagel nach einem der vorhergehenden Ansprüche,
dadurch **gekennzeichnet,**
daß das als separates Teil ausgebildete Befestigungsteil (8) und der Marknagel (4) so ausgebildet und aneinander befestigbar sind, daß sie sowohl für die rechten als auch für die linken Gliedmaßen verwendbar sind.

6. Marknagel nach einem der vorhergehenden Ansprüche,
dadurch **gekennzeichnet,**
daß das dem Befestigungsteil (8) zugewandte Ende des Hohlnagels (4) mit verstärkter Wandung ausgebildet ist.

7. Marknagel nach einem der vorhergehenden Ansprüche,
dadurch **gekennzeichnet,**
daß das Befestigungsteil (22) ein Distanzstück (21) umfaßt, das mit dem Hohlnagel (4) drehfest verbindbar ist, wobei das Distanzstück (21) als Hülse ausgebildet ist, die eine Kronenverzahnung (23) und mit dem Hohlnagel (4) über eine durch die Hülse verlaufende Schraube (24) verbunden ist.

## Claims

1. A medullary pin for intermedullary use in the healing of bone damage, in particular of fractures of tubular bones, the pin being insertable into a medullary cavity (3) of the bone and also securable at both ends to the bone (1) by fixation means (9, 11), wherein one end of the medullary pin (4) is formed as a fastening part (8) fixable to the outer side of the bone (1) by means of screws (9, 15) and wherein the medullary pin is formed as a hollow pin (4) which has an inner cavity (5) which is open at both ends,
characterised in that the fastening part (8, 22) is formed by a wall part of the hollow pin (4) or by a separate part which is connectable to the hollow pin (4); and in that the open end of the hollow pin (4) lying opposite to the fastener part (8, 22) has an internal or external thread (10) which can be screwed to a screw (11) with an external or internal thread (10) for the fixation of the hollow pin (4) inserted into the medullary cavity (3) from the outside, wherein the head of the screw (11) contacts the bone (1) from the outside, in particular via a washer (12) thereby preventing the head breaking into the bone (1).

2. Medullary pin (4) for intermedullary use in the healing of bone damage, in particular of fractures of tubular bones, the pin being insertable into a medullary cavity (3) of the bone and also securable at both ends to the bone (1) by fixation means (9, 11), wherein one end of the medullary pin (4) is formed as a fastening part (8) fixable to the outer side of the bone (1) by means of screws (9, 15) and wherein the medullary pin is formed as a hollow pin (4) which has an inner cavity (5) which is open at both ends, characterised in that the fastening part (8, 22) is formed by a wall part of the hollow pin (4) or by a separate part which is connectable to the hollow pin (4); and in that a terminal cap (6) formed as a point is screwably secured to the open end of the hollow pin (4) lying opposite to the fastening part (8, 22) and preferably has lateral outlet openings (17) which, when the terminal cap (16) is screwed onto the end of the hollow pin (4), communicate via channels (18) with the inner space (5) of the hollow pin (4) in which spike wires (19) are displaceably arranged, with the ends of the spike wires adjacent the terminal cap (16) being displaceable via the channels (18) through the outlet openings (17) and with their ends adjacent the fastening part (18) being securable to the latter.

3. Medullary pin in accordance with claim 2, characterised in that the terminal cap (16) has two outlet openings (17) and two spike wires (19) are arranged in the inner space (5) of the hollow pin (4).

4. Medullary pin in accordance with claim 2 or claim 3, characterised in that a device for securing the spike wires (19), which is in particular formed as a clamping screw (20), is arranged at the securing part (8).

5. Medullary pin in accordance with one of the preceding claims, characterised in that the fastener part (8), which is formed as a separate part, and the medullary pin (4) are so formed and so securable to one another that they can be used both for the right-hand limbs and also for the left-hand limbs.

6. Medullary pin in accordance with one of the preceding claims, characterised in that the end of the hollow pin (4) adjacent the fastener part (8) is formed with a reinforced wall.

7. Medullary pin in accordance with one of the preceding claims, characterised in that the fastener part (22) includes a spacer piece (21) which can be rotationally fixedly connected to the hollow pin (4), with the spacer piece (21) being formed as a sleeve having a toothed crown (23) and connected to the hollow pin (4) via a screw (24) extending through the sleeve.

## Revendications

1. Broche ou clou destiné à l'application intramédullaire pour le traitement de traumatismes osseux, en particulier des fractures d'os tubulaires, que l'on peut mettre en place dans un canal médullaire (3) de l'os et fixer aux deux extrémités sur l'os (1) par des moyens de fixation (9, 11), une extrémité du clou intramédullaire (4) étant réalisée sous forme d'une pièce de fixation (8) susceptible d'être fixée sur la face extérieure de l'os (1) au moyen de vis (9, 15), et le clou intramédullaire étant réalisé sous forme d'un clou creux (4) qui possède une chambre intérieure (5) ouverte aux deux extrémités, caractérisé en ce que la pièce de fixation (8, 22) est formée par une partie de paroi du clou creux (4) ou par une partie séparée qui peut être reliée au clou creux (4), et en ce que l'extrémité ouverte du clou creux (4) située en vis-à-vis de la pièce de fixation (8, 22) présente un taraudage ou un filetage (10) qui peut être vissé avec une vis (11) présentant un filetage ou un taraudage (10) pour la fixation depuis l'extérieur du clou creux (4) mis en place dans le canal médullaire (3), la tête de la vis (11) s'appuyant en particulier via une rondelle (12) depuis l'extérieur contre l'os (1) en empêchant ainsi un enfoncement de la tête dans l'os (1).

2. Clou (4) destiné à l'application intramédullaire pour le traitement de traumatismes osseux, en particulier des fractures d'os tubulaires, que l'on peut mettre en place dans un canal médullaire (3) de l'os (1) et fixer aux deux extrémités sur l'os (1) par des moyens de fixation (9, 11), une extrémité du clou intramédullaire (4) étant réalisée sous forme d'une pièce de fixation (8) susceptible d'être fixée sur la face extérieure de l'os (1) au moyen de vis (9, 15), et le clou intramédullaire sous forme d'un clou creux (4) qui possède une chambre intérieure (5) ouverte aux deux extrémités, caractérisé en ce que la pièce de fixation (8, 22) est formée par une partie de paroi du clou creux (4) ou par une partie séparée qui peut être reliée au clou creux (4), et en ce qu'à l'extrémité ouverture du clou creux (4) située en vis-à-vis de la pièce de fixation (8, 22) est fixé par vissage un capuchon de fermeture (16) réalisé sous forme d'une pointe, qui présente de préférence des ouvertures de sortie latérales (17) qui, lorsque le capuchon de fermeture (16) est vissé à l'extrémité du clou creux (4), sont en liaison via des canaux (18) avec la chambre intérieure (5) du clou creux (4), dans laquelle sont agencés en déplacement des fils de bardage (19) dont les extrémités orientées vers le capuchon de fermeture (16) peuvent être déplacées via les canaux (18) à travers les ouvertures de sortie (17), et dont les extrémités orientées vers la pièce de fixation (8) peuvent être fixées sur celle-ci.

3. Clou intramédullaire selon la revendication 2, caractérisé en ce que le capuchon de fermeture (16) présente deux ouvertures de sortie (17), et en ce que deux fils de bardage (19) sont agencés dans la chambre intérieure (5) du clou creux (4).

4. Clou intramédullaire selon l'une ou l'autre des revendications 2 et 3, caractérisé en ce qu'il est prévu sur la pièce de fixation (8) un dispositif pour fixer les fils de bardage (19), réalisé en particulier sous forme de vis de serrage (20).

5. Clou intramédullaire selon l'une quelconque des revendications précédentes, caractérisé en ce que la pièce de fixation (8) réalisée sous forme d'une partie séparée et le clou intramédullaire (4) sont réalisés et susceptibles d'être fixés l'un sur l'autre de telle sorte qu'ils peuvent être appliqués tant pour les membres de droite que pour les membres de gauche.

6. Clou intramédullaire selon l'une quelconque des revendications précédentes, caractérisé en ce que l'extrémité du clou creux (4) orientée vers la pièce de fixation (8) est réalisée avec une paroi renforcée.

7. Clou intramédullaire selon l'une quelconque des revendications précédentes, caractérisé en ce que la pièce de fixation (22) comporte un élément d'écartement (21) qui peut être relié solidairement en rotation au clou creux (4), l'élément d'écartement (21) étant réalisé sous forme d'une douille qui présente une denture couronnée (23) et qui est reliée au clou creux (4) via une vis (24) s'étendant à travers la douille.
